# EUROPÄISCHE PATENTSCHRIFT

(11) **EP 3 009 111 B1**
(45) Veröffentlichungstag und Bekanntmachung des Hinweises auf die Patenterteilung: **30.01.2019**
(21) Anmeldenummer: 15002936.1
(22) Anmeldetag: 15.10.2015
(51) Int. Cl.: A61F 5/01, A61F 5/058

(54) **HÜFTGELENKENTLASTUNGSORTHESE**
HIP JOINT RELIEF ORTHOSIS
ORTHESE DE DECHARGE DE LA HANCHE

(30) Priorität: 15.10.2014 DE 202014008160 U
(43) Veröffentlichungstag der Anmeldung: 20.04.2016
(73) Patentinhaber: Spörer AG, 85049 Ingolstadt (DE)
(72) Erfinder: Reiter-Haringer, Michael, 83373 Taching am See (DE); Bengs, Oliver, 83308 Trostberg-Mögling (DE); Schuhbeck, Daniel, 83569 Vogtareuth (DE)
(74) Vertreter: Liebl, Thomas

(56) Entgegenhaltungen:
- DE-A1-102010 025 578
- DE-C1- 19 500 271
- US-B1- 6 540 703
- BERGMANN G ET AL: "Evaluation of ischial weight-bearing orthoses, based on in-vivo hip joint force measurements", CLINICAL BIOMECHANICS, BUTTERWORTH SCIENTIFIC LTD, GUILDFORD, GB, Bd. 9, Nr. 4, 1. Juli 1994 (1994-07-01), Seiten 225-234, XP026180947, ISSN: 0268-0033, DOI: 10.1016/0268-0033(94)90003-5 [gefunden am 1994-07-01]

## Beschreibung

Die Erfindung betrifft eine Hüftgelenkentlastungsorthese nach dem Oberbegriff des Anspruchs 1.

Eine derartige Hüftgelenkentlastungsorthese dient der Entlastung des Hüftgelenkes bei Morbus-Perthes. Der Morbus-Perthes ist eine orthopädische Kinderkrankheit, die insbesondere bei vier- bis zwölfjährigen Kindern auftritt und bei der es aufgrund von Durchblutungsstörungen zu einem Absterben von Knochengewebe im Hüftkopf kommt. Die erkrankten Kinder entwickeln regelmäßig ein Schonhinken, Knieschmerzen bzw. Hüftgelenkrotationseinschränkungen. Im Frühstadium führt die Erkrankung zu einer Gelenkreizung mit Gelenkergüssen, so dass Ähnlichkeit mit rheumatischen Erkrankungen besteht. Im weiteren Verlauf tritt dann regelmäßig ein Zusammensintern der Hüftkopfkugel auf, was oft auch mit einem seitlichen Auswandern aus dem Gelenkkugellager im Becken verbunden ist. Später kommt es dann zu einer bleibenden Verformung von Kopf und Pfanne mit einer entsprechenden Bewegungsstörung und einem verkürzt bleibenden Bein. Der frühe Verschleiß des Hüftgelenkes ist durch diese Krankheit vorbestimmt.

Sobald ein Morbus-Perthes erkannt worden ist, wird im Rahmen der Therapie versucht, den geschwächten Hüftkopf zu entlasten und das Auftreten von Deformationen zu verhindern. Bei der Hüftgelenkentlastung wird versucht, das Hüftgelenk in eine Stellung zu bringen, die sich günstig auf die Heilung auswirkt.

Beispielsweise ist zur Hüftgelenkentlastung der Einsatz einer sogenannten Thomasschiene, auch Thomas-Splint genannt, bekannt, die eine den Oberschenkel im Übergangsbereich zum Hüftgelenk umgreifende Oberschenkel-Fassung aufweist, die als Tubersitz ausgebildet ist und sich in der Orthesen-Gebrauchsstellung mittelbar über die Weichteile am Sitzbein, insbesondere am Sitzbeinhöcker, abstützt. Von dieser Oberschenkel-Fassung laufen rechts und links entlang des Beines zwei Metallbänder nach unten, die dort in einem fußseitigen Gehbügel enden. Die Schiene wird insgesamt mit Manschetten am Bein gehalten. Bei angelegter Thomasschiene schwebt die Ferse des betroffenen Beins auch bei Belastung einige Zentimeter über dem Boden. Deshalb muss auf dem anderen Bein die Schuhsohle entsprechend erhöht werden.

Desweiteren wird zur Hüftgelenkentlastung bei einem Morbus-Perthes oftmals auch die sogenannte Mainzer Orthese eingesetzt, die eine Weiterentwicklung der Thomasschiene ist und ebenfalls der Entlastung des Hüftgelenks dient. Von der oben beschriebenen Thomasschiene unterscheidet sich die Mainzer Orthese insbesondere durch den sogenannten PTF (Pelvis-Trochanter-Femur)-Ringschaft, bei dem durch entsprechende Aussparungen das Trochantermassiv entlastet wird und der Druck auf das Hüftgelenk vermindert werden soll. Desweiteren unterscheidet sich die Mainzer Orthese von der Thomasschiene durch die abduzierte sowie innenrotierte Zwangshaltung des Beines. Das Bein schwebt hier nicht vollständig in der Luft, sondern wird mittels des Schuhs an einer Stahlfeder befestigt, die für eine Extension und Innenrotation des Beines sorgt, aber eine Belastung verhindert. Am gesunden Bein ist eine Schuherhöhung von 6 bis 8 cm erforderlich. Anders als bei der Thomasschiene ist zudem die Metallschiene hier dauerhaft starr ausgebildet und kann in diese kein Kniegelenk integriert werden, was bei der Thomasschiene bei größeren Kinder manchmal vorgesehen wird.

Ein anderes Therapiemittel, das in Verbindung mit einem Morbus-Perthes eingesetzt wird, ist ein Beckenbeingipsverband in Abduktions- oder Entlastungsstellung, der Einsatz eines Spreizgipses (Petrie-Cast), bei dem es sich ebenfalls um einen speziellen Gipsverband handelt oder der sogenannte Braad-Gips, bei der Unter- und Oberschenkel der Patienten so eingegipst werden, dass das Knie spitzwinklig gebeugt ist. Zudem ist in Verbindung mit dem Morbus-Perthes auch der Einsatz der sogenannten Snyder-Schlinge bekannt. Hierbei handelt es sich um einen Gurt, der über eine Schulter geführt wird und so lang ist, dass der Unterschenkel meist in Höhe des Sprunggelenks eingehängt werden kann. Der Fuß selbst bleibt unbeschuht. Wenn das Kind aufrecht steht, sollte der Unterscherschenkel waagrecht hängen. Die Kinder gehen dann an Unterarm-Gehstützen.

Aus der DE 195 00 271 C1 ist weiter eine Orthese für die Behandlung von Morbus-Perthes bei Kleinkindern mit einem das Becken des Patienten umfassenden Korsett, an dem Oberschenkel anlegbaren Hülsen, die jeweils durch eine Verbindungsstange an das Korsett angeschlossen sind, und einem die Hülsen miteinander verbindenden, gelenkig an diese angeschlossenen Spreizstab bekannt. Die Verbindungsstangen weisen jeweils ein Gelenk auf, welches Schrittbewegungen zulässt, wobei die Verbindungsstangen mit einer durch die Länge des Spreizstabes vorgegebenen, nach außen gerichteten, V-förmigen Abspreizung an das Korsett anschließen. Der Spreizstab ist weiter beidendig an die Rückseite der Hülsen angeschlossen, wobei das Gelenk einer der beiden Verbindungsstangen als Scharnier ausgebildet ist, welches lediglich eine Schwenkbewegung in Schrittrichtung ermöglicht. Das Gelenk der anderen Verbindungsstange ist dagegen als Schwenk- und Drehgelenk ausgeführt, welches eine Schwenkbewegung in Schrittrichtung und außerdem eine begrenzte, nach innen gerichtete Drehbewegung zulässt.

Aus der US 6,540,703 B1 ist eine Hüftabduktionsorthese bekannt, mit der postoperativ ein Auskugeln des Hüftgelenks vermieden werden soll. Konkret umfasst diese Hüftabduktionsorthese einen Beckengurt, an den über eine Verbindung mit einem Schwenkgelenk eine den Oberschenkel umschließende Manschette angeschlossen ist.

Weiter ist aus der DE 10 2010 025 578 A1 eine Stütze für eine untere Extremität bekannt, die eine Oberschenkelstütze und eine Unterschenkelstützte aufweist, die mit einem Gelenk miteinander verbunden sind. An die Unterschenkelstütze ist eine Stützenbefestigung für die als gehbelastbare Stiefelanordnung ausgebildete Stütze vorgesehen. Zwischen der Stützenbefestigung und der Unterschenkelstütze ist eine Dämpfungseinrichtung vorgesehen, die zur Kraftübertragung miteinander gekoppelt sind und deren Dämpfungskraft einstellbar ist.

Weitere Therapiemaßnahmen sind Unterarm-Gehhilfen bzw. das Sitzen im Rollstuhl.

Wie die oben gemachten Ausführungen der Therapiemöglichkeiten zeigen, bedingen sämtliche Therapiemaßnahmen bzw. Therapiehilfsmittel eine deutliche Einschränkung der Bewegungsfreiheit des jeweiligen Patienten. Zudem erfordern sämtliche Maßnahmen eine oftmalige, intensive und aufwendige orthopädietechnische bzw. orthopädisch-medizinische Betreuung und Begleitung.

Demgegenüber ist es Aufgabe der vorliegenden Erfindung, eine Hüftgelenkentlastungsorthese zu schaffen, die bei einer ausgezeichneten Hüftgelenkentlastung dem Patienten gleichzeitig größtmögliche persönliche Freiräume gewährleistet, insbesondere im Hinblick auf die Frequenz der orthopädietechnischen bzw. medizintechnischen Versorgung.

Diese Aufgabe wird gelöst mit den Merkmalen des Schutzanspruchs 1. Vorteilhafte Ausgestaltungen sind Gegenstand der darauf rückbezogenen Unteransprüche.

Gemäß Anspruch 1 wird eine Hüftgelenkentlastungsorthese mit einer, bezogen auf die Orthesen-Gebrauchsstellung, oberen, den Oberschenkel wenigstens bereichsweise im Übergangsbereich zum Hüftgelenk umgreifenden Oberschenkel-Fassung vorgeschlagen, die als Tubersitz ausgebildet ist und sich in der Orthesen-Gebrauchsstellung mittelbar über die Weichteile des Körpers am Sitzbein, insbesondere am Sitzbeinhöcker, abstützt. Erfindungsgemäß ist vorgesehen, dass die Hüftgelenkentlastungsorthese weiter eine, bezogen auf die Orthesen-Gebrauchsstellung untere, von der oberen Oberschenkel-Fassung beabstandete Oberschenkel-Fassung aufweist, die den Oberschenkel im Übergangsbereich zum Kniegelenk wenigstens bereichsweise umgreift und sich in der Orthesen-Gebrauchsstellung mittelbar über die Weichteile des Körpers am unteren verdickten Ende des Oberschenkelknochens, insbesondere an den beiden Femurkondylen, abstützt. Weiter ist erfindungsgemäß wenigstens eine Verstelleinrichtung vorgesehen, die einerseits an der oberen Oberschenkel-Fassung und andererseits an der unteren Oberschenkel-Fassung angreift und mittels der, zur Einstellung einer, eine definierte Hüftgelenkentlastung bewirkenden Verspannkraft zwischen dem unteren verdickten Ende des Oberschenkel-Knochens und dem Sitzbein in der Orthesen-Gebrauchsstellung, der Abstand zwischen der oberen Oberschenkel-Fassung und der unteren Oberschenkel-Fassung veränderbar und einstellbar ist.

Mit der erfindungsgemäßen Lösung wird eine besonders vorteilhafte Hüftgelenkentlastung erzielt, weil die jeweilige Verspannkraft der Orthese zwischen dem unteren verdickten Ende des Oberschenkel-Knochens und dem Sitzbein in der Orthesen-Gebrauchsstellung jeweils individuell für jeden Patienten eingestellt werden kann. Wenngleich die Einstellung der jeweils erforderlichen Verspannkraft und damit der jeweiligen Hüftgelenkentlastung bevorzugt vom Orthopädietechniker bzw. Orthopäden vorgenommen wird, kann unter Umständen diese Einstellung auch vom Patienten bzw. von den Eltern des Patienten selbst vorgenommen werden.

Mit der erfindungsgemäß vorgesehenen wenigstens einen Verstelleinrichtung kann zudem mit ein und derselben Orthese die entsprechende Verspannkraft jeweils auf einfache Weise verändert bzw. nachjustiert werden, was zum Beispiel bei wachstumsbedingten Veränderungen am Patienten erforderlich ist und somit die gewünschte Verspannkraft bzw. Hüftgelenkentlastung einfachst an jeweils vorherschende Bedingungen angepasst werden.

Ein wesentlicher Vorteil ist zudem, dass mit der erfindungsgemäßen Lösung der natürliche Bewegungsablauf des Patienten, insbesondere im Hinblick auf die 3-dimensionalen Rotationsbewegungen im Hüftgelenkbereich bei einer normalen Gangfolge, nicht eingeschränkt wird und die natürliche Bewegung in vollem Umfang erhalten bleibt.

Die wenigstens eine Verstelleinrichtung kann grundsätzlich auf unterschiedlichste Weise ausgebildet werden. Besonders bevorzugt ist diese Verstelleinrichtung als Linearverstelleinrichtung ausgebildet, mittels der der Abstand zwischen den beiden Oberschenkel-Fassungen bzw. die Verspannkraft auf einfache und funktionssichere Weise linear bzw. geradlinig eingestellt werden kann.

Besonders bevorzugt ist die Verstelleinrichtung durch eine Zylinder-Kolben-Einheit mit einem in einem Zylinder geführten Kolben sowie mitsamt dem Kolben zugeordneter Kolbenstange gebildet, wobei der Kolben zur Verlagerung in eine vorgegebene Abstützposition mit einem Arbeitsmedium beaufschlagbar ist. Der Zylinder ist dabei mit einer ersten der beiden Oberschenkel-Fassung und die Kolbenstange mit einer zweiten der beiden Oberschenkel-Fassungen verbunden. Mit einer derartigen Zylinder-Kolben-Einheit, die durch entsprechendes Zuführen bzw. Abführen eines Arbeitsmediums betätigbar ist, lässt sich der Abstand zwischen den beiden Oberschenkelfassungen und damit die Verspannkraft zwischen dem unteren verdickten Ende des Oberschenkelknochens und dem Sitzbein auf besonders funktionssichere Weise verändern bzw. einstellen, wobei die einmal eingenommene Abstützposition dann zuverlässig gehalten wird.

Die Zylinder-Kolben-Einheit ist bevorzugt als einfach wirkende Verstelleinrichtung ausgebildet, bei der das Arbeitsmedium in einen Arbeitsraum des Zylinders einströmt und den Kolben gegen die Kraft eines Federelementes in eine definiert vorgegebene Kolbenposition verlagert. Ein derartiger Aufbau ist herstellungstechnisch einfach und in der Anwendung funktionssicher. Grundsätzlich könnte die Zylinder-Kolben-Einheit aber auch als doppelt wirkende Verstelleinrichtung mit beidseits des Kolbens beaufschlagbaren Arbeitsräumen ausgebildet sein, wenngleich dies insgesamt aufwendiger ist.

In Verbindung mit der als einfach wirkende Verstelleinrichtung ausgebildeten Zylinder-Kolben-Einheit ist es von besonderem Vorteil, wenn in einem, dem Arbeitsraum mit Bezug zum Kolben gegenüberliegenden Zylinderraum wenigstens eine Be- und/oder Entlüftungsöffnung vorgesehen ist, um das unerwünschte Aufbauen eines Überdrucks zuverlässig zu vermeiden.

Die Zylinder-Kolben-Einheit könnte grundsätzlich durch einen Hydraulikzylinder mit einem Hydrauliköl oder einem vergleichbaren unkomprimierbaren Medium gebildet sein, der jedoch insgesamt eine relativ starre und träge Verbindung zwischen den beiden Oberschenkelfassungen bewirkt und bei stoßartigen Beinbelastungen zu einer relativ hohen Kraftbelastung im Bereich des Sitzbeins bzw. im Bereich des unteren verdickten Ende des Oberschenkelknochens führt, was von den Patienten ggf. als unangenehm empfunden wird. Ein solcher Aufbau ist somit insbesondere dann vorteilhaft, wenn eine insgesamt relativ starre bzw. träge Dämpferverbindung mit der Verstelleinrichtung ausgebildet werden soll. Gemäß einer hierzu alternativen Ausführungsform kann die Zylinder-Kolben-Einheit aber auch durch einen Pneumatikzylinder mit einem komprimierbaren Gas, insbesondere mit Luft, als Arbeitsmedium gebildet sein, weil hier zwar ebenfalls eine relativ starre und feste Verbindung zwischen den beiden Oberschenkelfassungen hergestellt wird, jedoch bei stoßartigen Belastungen durch das komprimierbare Gas eine gewisse Abfederung der Stoßbelastung erfolgen kann, was die Belastung auf das Sitzbein bzw. das verdickte Ende des Oberschenkelknochens reduziert.

Grundsätzlich kann es ausreichend sein, eine einzige Verstelleinrichtung zwischen den beiden Oberschenkel-Fassungen vorzusehen. Für eine vorteilhafte, gleichmäßige Abstützung und insbesondere auch Verlagerungsmöglichkeit zur Einstellung des Abstandes zwischen den beiden Oberschenkelfassungen ist jedoch bevorzugt der Einsatz mehrerer Verstelleinrichtungen vorgesehen, die in der Orthesen-Gebrauchsstellung in Umfangsrichtung um den Oberschenkel herum voneinander beabstandet sind, und zwar insbesondere dergestalt, dass zwei in Umfangsrichtung diametral voneinander beabstandete Verstelleinrichtungen vorgesehen sind, die zum Beispiel an der Innenseite bzw. an der Außenseite liegen.

Gemäß einer besonders konkreten Ausführungsform weist der Zylinder der Zylinder-Kolben-Einheit wenigstens ein Anschlusselement, insbesondere ein Anschlussventil, für eine Mediumleitung auf, über das das Arbeitsmedium in den Arbeitsraum einbringbar und/oder ausbringbar ist. Beispielsweise kann das Einbringen und das Ausbringen des Arbeitsmediums, zum Beispiel eines komprimierbaren Gases, wie beispielsweise Luft, als Arbeitsmedium über ein und denselben Anschluss erfolgen. Grundsätzlich könnte aber auch vorgesehen sein, dass Arbeitsmedium über einen ersten Anschluss in den Arbeitsraum einzubringen und eventuell über einen zweiten Anschluss aus dem Arbeitsraum abzuführen, sofern das Abführen überhaupt erforderlich sein sollte. Hierfür wären dann die Anschlüsse bzw. Anschlusselemente, die bevorzugt durch Anschlussventile gebildet sind, entsprechend von einer Steuer- und/oder Regeleinrichtung anzusteuern.

Die Mediumleitung selbst ist bevorzugt mit einem Reservoir für das Arbeitsmedium gekoppelt, aus dem heraus das Arbeitsmedium über eine entsprechend vorgesehene Fördereinrichtung gesteuert bzw. geregelt mittels einer Steuer- und/oder Regeleinrichtung in einer definiert vorgegebenen Weise in den Arbeitsraum über das wenigstens eine Anschlusselement einbringbar. Damit kann eine definiert vorgegebene Einstellung des Abstandes zwischen oberen Oberschenkel-Fassung und der unteren Oberschenkel-Fassung und damit eine definierte Hüftgelenkentlastung bewirkende Verspannkraft zwischen dem unteren verdickten Ende des Oberschenkelknochens und dem Sitzbein eingestellt werden.

Gemäß einer weiteren besonders bevorzugten Ausgestaltung ist die Mediumleitung bevorzugt lösbar mit dem zylinderseitigen Anschlusselement verbunden, so dass die Hüftgelenkentlastungsorthese auch ohne Schlauchleitungen etc. verwendet und getragen werden kann. Es versteht sich, dass im abgekoppelten Zustand der Mediumleitung selbstverständlich das wenigstens eine Anschlusselement dicht verschlossen sein muss.

Um die Hüftgelenkentlastungsorthese insgesamt für den Patienten vom Tragkomfort her angenehmer zu gestalten bzw. dieser Orthese eine gewisse innere Beweglichkeit zuzugestehen, die sich nicht negativ auf die voreingestellte Hüftgelenkentlastung auswirkt, kann die wenigstens eine Verstelleinrichtung an der unteren und/oder oberen Oberschenkelfassung gelenkig gelagert sein. Diese gelenkige Lagerung erfolgt insbesondere mittels eines Kugelgelenkes.

Gemäß einer besonders bevorzugten konkreten Ausgestaltung ist die obere Oberschenkel-Fassung als, vorzugsweise geschlossene, Ringfassung ausgebildet, die sich, bezogen auf die Orthesen-Gebrauchsstellung, am oberen Fassungsrand mit einem der Oberschenkelinnenseite zugeordneten Teilbereich in Richtung zum Sitzbein hin nach außen krümmt oder wölbt und einen Sitzbein-Abstützbereich ausbildet. Mit einer derartigen konkreten Ausgestaltung wird ein besonders vorteilhafter und aufgrund der gekrümmten, weichen Ausgestaltung vom Patienten als angenehm empfundener Tubersitz ausgebildet.

Desweiteren kann sich die obere Oberschenkel-Fassung bezogen auf die Orthesen-Gebrauchsstellung, am oberen Fassungsrand mit einem der Oberschenkelaußenseite zugeordneten Teilbereich über den Bereich des großen Rollhügels des Oberschenkelknochens hinaus erstrecken. Dadurch ist sichergestellt, dass sich die obere Oberschenkel-Fassung lediglich am Sitzbein abstützt, nicht dagegen im Bereich des großen Rollhügels des Oberschenkelknochens, was vorteilhaft für die Hüftgelenkentlastung ist.

Desweiteren kann sich die obere Oberschenkel-Fassung vom, bezogen auf die Orthesen-Gebrauchsstellung oberen Fassungsrand ausgehend mit einem zylinderförmigen Schaftbereich bis über den Bereich des kleinen Rollhügels des Oberschenkelknochens hinaus in den Bereich des Oberschenkelschaftes erstrecken. Mit einem derartigen zylinderförmigen Schaftbereich wird eine besonders vorteilhafte und vom Patienten bezüglich des Tragekomforts als angenehm empfundene, wenig einschneidende Abstützung des oberen Oberschenkelbereichs erzielt.

Gemäß einer weiteren besonders bevorzugten Ausgestaltung wird vorgeschlagen, dass die untere Oberschenkelfassung als, vorzugsweise öffenbare, Ringfassung ausgebildet ist, die, bezogen auf die Orthesen-Gebrauchsstellung, zwei voneinander in Umfangsrichtung beabstandete sowie nach unten abragende lappenartige Fortsätze aufweist, die sich in der Orthesen-Gebrauchsstellung mittelbar über die Weichteile des Körpers jeweils an einem der Femurkondylen abstützen. Mit derartigen nach unten abragenden lappenartigen Fortsätzen wird auf einfache und funktionssichere Weise sichergestellt, dass sich die untere Oberschenkelfassung genau dort abstützt, wo dies gewünscht ist und somit keine Fehlbedienung bzw. Fehlfunktionen auftreten können. Dies trägt wesentlich dazu bei, eine optimierte Hüftgelenkentlastung zu schaffen.

Besonders bevorzugt ist eine Ausgestaltung, bei der zwei Verstelleinrichtungen, insbesondere zwei Zylinder-Kolben-Einheiten als Verstelleinrichtungen, vorgesehen sind, von denen jeweils eine im Bereich eines der lappenartigen Fortsätze angreift und dort angebunden ist. Die Anbindung erfolgt dabei bevorzugt gelenkig, zum Beispiel über Kugelgelenke. Weiter erstrecken sich die Verstelleinrichtungen dann bevorzugt von den lappenseitigen Anbindungsbereichen ausgehend im Wesentlichen geradlinig nach oben zu ihrer, vorzugsweise gelenkigen, Anbindungsstelle an der oberen Oberschenkel-Fassung.

Damit ergibt sich insgesamt ein kompakter Aufbau der Hüftgelenkentlastungsorthese mit ausreichender Abstützfunktion, wobei die beiden Verstelleinrichtungen des weiteren einfach und funktionssicher in die jeweilige Abstütz- und Verspannposition überführt werden können.

Gemäß einer weiteren besonders bevorzugten Ausgestaltung wird vorgeschlagen, dass sich die untere Oberschenkel-Fassung, bezogen auf die Orthesen-Gebrauchsstellung, von den beiden lappenartigen Fortsätzen ausgehend mit einem zylinderförmigen Schaftbereich nach oben in Richtung zum mittleren Oberschenkelbereich erstreckt. Auch hier wird wiederum mit dem zylinderförmigen Schaftbereich eine vorteilhafte und vom Patienten als angenehm empfundene flächige Abstützung sichergestellt und dadurch ein Einschnüren bzw. Einschneiden der unteren Oberschenkel-Fassung zuverlässig vermieden.

Die untere Oberschenkel-Fassung ist bevorzugt als öffenbare Ringfassung ausgebildet, die sich im Bereich des zylinderförmigen Schaftbereiches in Umfangsrichtung gegebenenfalls überlappende und/oder einander zugeordnete Teilbereiche aufweist, an denen wenigstens ein Fixierelement angeordnet ist. Das Ausbilden der unteren Oberschenkel-Fassung als öffenbare Ringfassung erleichtert das Anlegen der Hüftgelenkentlastungsorthese insgesamt. Zudem kann dann die Anpresskraft des zylinderförmigen Schaftbereiches an den zugeordneten Oberschenkelbereich individuell festgelegt werden.

Das wenigstens eine Fixierelement ist bevorzugt durch einen Klettverschluss bzw. eine Klettverschlussanordnung gebildet, der bzw. die zum Beispiel ein an einem ersten Teilbereich festgelegtes Klettband aufweist, das durch eine am anderen, zweiten Teilbereich gelagerte Öse hindurchführbar und zur Festlegung wieder zurück zu dem Befestigungsbereich am ersten Teilbereich geführt werden kann. Mit einem derartigen Klettverschluss kann eine funktionssichere Festlegung erzielt werden, die zudem einfach zu bedienen ist.

Die Erfindung wird nachfolgend anhand einer Zeichnung lediglich beispielhaft näher erläutert.

Es zeigen:
- Fig. 1: eine perspektivische Vorderansicht einer beispielhaften Ausführungsform einer erfindungsgemäßen Hüftgelenkentlastungsorthese,
- Fig. 2: schematisch den Knochenapparat im Bereich des Hüftgelenkes mitsamt zugeordnetem Oberschenkelknochen,
- Fig. 3: die Hüftgelenkentlastungsorthese gemäß Fig. 1 mit schematisch zugeordnetem Knochenapparat gemäß Fig. 2,
- Fig. 4: eine schematische, perspektivische und vergrößerte Detailansicht der oberen Oberschenkel-Fassung,
- Fig. 5: eine gegenüber der Fig. 4 in etwa um 45° gedrehte Darstellung der oberen Oberschenkel-Fassung,
- Fig. 6: eine schematische, perspektivische und vergrößerte Detaildarstellung der unteren Oberschenkel-Fassung,
- Fig. 7: schematisch eine Prinzipskizze der als einfach wirkender Pneumatikzylinder ausgebildeten Verstelleinrichtung.

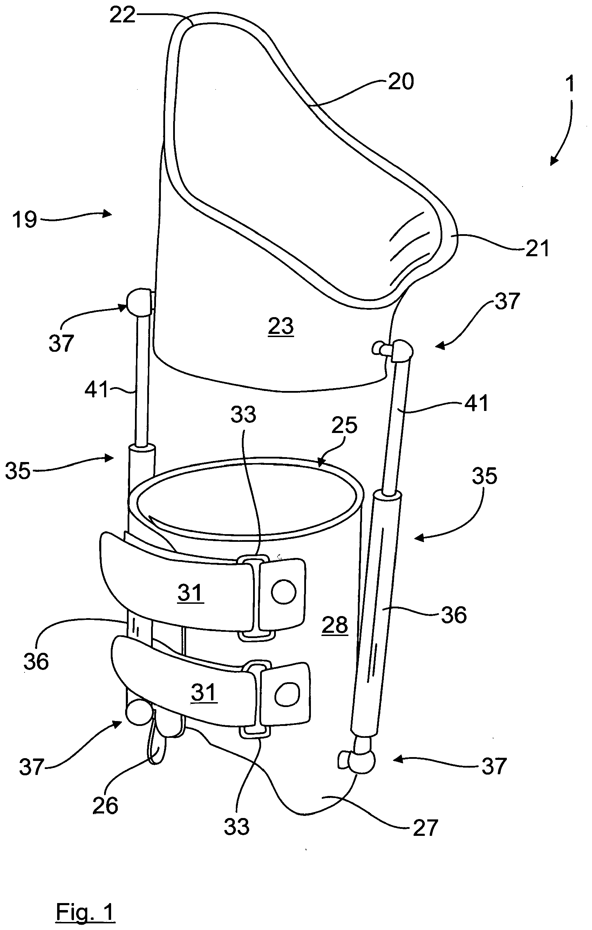

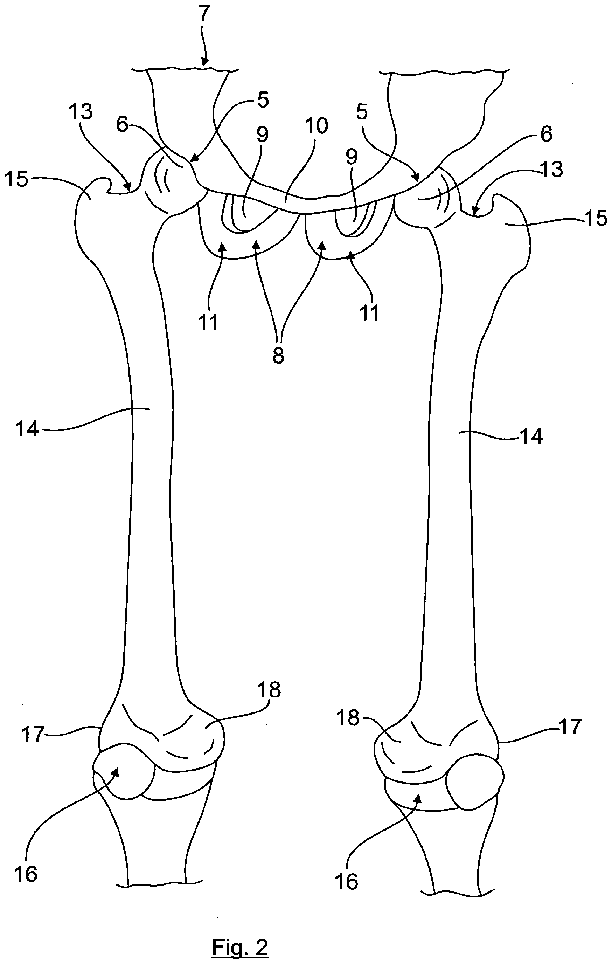

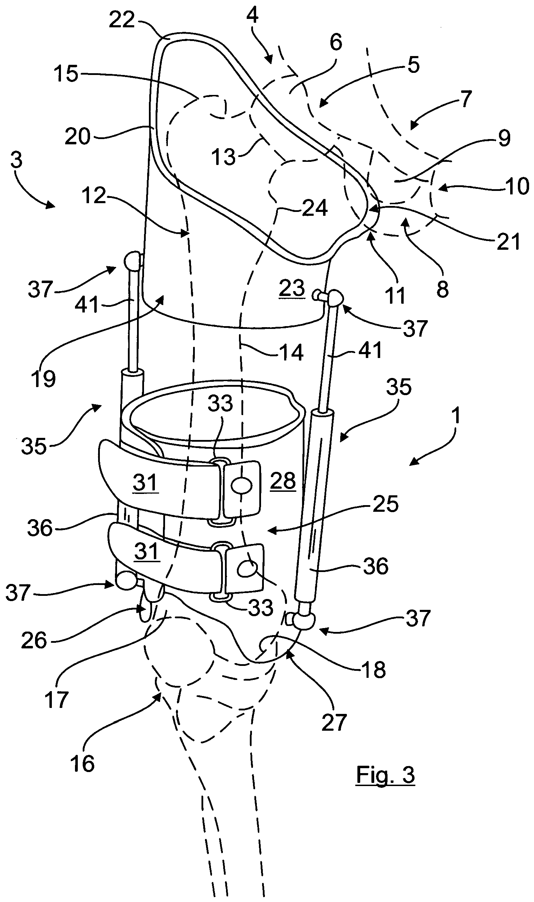

In der Fig. 1 ist schematisch und beispielhaft eine Ausführungsform einer erfindungsgemäßen Hüftgelenkentlastungsorthese 1 gezeigt, deren Aufbau und Funktionsweise vor allem mit Bezug zur Fig. 3 anhand des im Detail in der Fig. 2 gezeigten Knochenapparates 2 erläutert wird. In der Fig. 3 ist die Hüftgelenkentlastungsorthese 1 somit in einer Orthesen-Gebrauchsstellung 3 gezeigt.

Die Hüftgelenkentlastungsorthese 1 kommt insbesondere bei einem Morbus-Perthes zum Einsatz, bei der eine Entlastung des Hüftgelenks 4 erforderlich ist, kann jedoch grundsätzlich auch für jedwede andere Therapie eingesetzt werden, bei der eine Hüftgelenkentlastung erforderlich ist.

Das Hüftgelenk 4 setzt sich insbesondere aus der Hüftpfanne 5 und dem darin in an sich bekannter Weise aufgenommenen Hüftkopf 6 zusammen.

Die Hüftpfanne 5 ist Bestandteil des Beckenknochens 7, der hier nicht weiter dargestellt ist, jedoch in seinem unteren Bereich links- und rechtsseitig jeweils das Sitzbein 8 aufweist, das das Hüftloch 9 umschließt und in das Schambein 10 übergeht. Das Sitzbein 8 weist jeweils an seiner Unterseite jeweils den Sitzbeinhöcker 11 (Tuber ischiadicum) auf.

Der Hüftkopf 6 ist dagegen jeweils Bestandteil eines Oberschenkelknochens (Femur) 12, wobei der Hüftkopf 6 über einen Schenkelhals 13 in den Oberschenkelschaft 14 übergeht. Im Bereich des Schenkelhalses 13 befindet sich noch der sogenannte große Rollhügel 15.

Am einem dem Hüftgelenk 4 gegenüberliegenden Ende geht der Oberschenkelknochen 12 in das Kniegelenk 16 über, wobei das untere Ende des Oberschenkelknochens 12 verdickt ausgebildet ist und zwei diese Verdickungen ausbildende Femurkondylen 17, 18 aufweist.

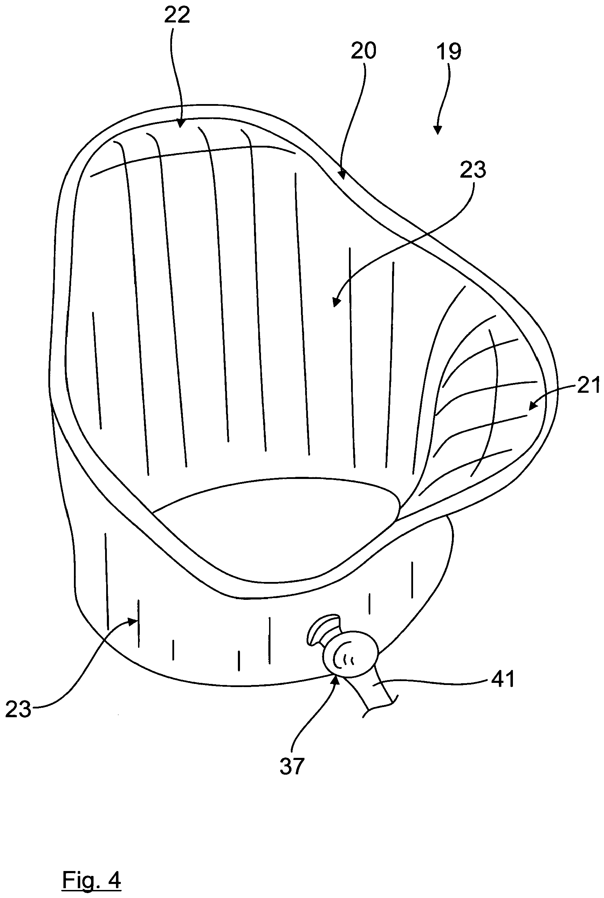

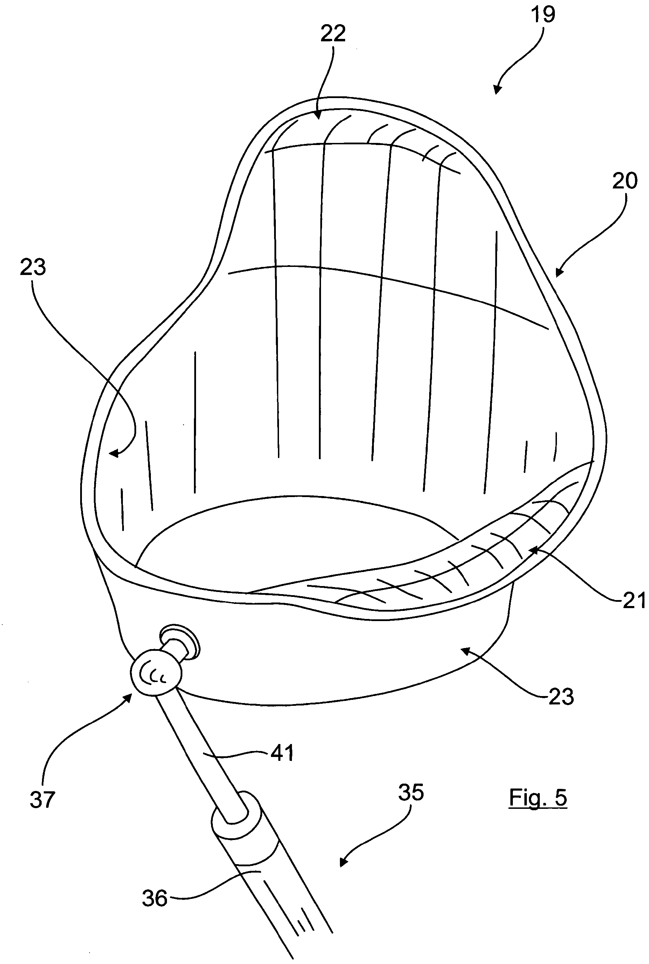

Wie dies nunmehr insbesondere aus der Zusammenschau der Fig. 1 und 3 mit der Fig. 2 ersichtlich ist, weist die hier beispielhaft dargestellte erfindungsgemäße Hüftgelenkentlastungsorthese 1 eine den Oberschenkel im Übergangsbereich zum Hüftgelenk ringförmig umgreifende und als Tubersitz ausgebildete, obere Oberschenkelfassung 19 auf, die sich in der Orthesen-Gebrauchsstellung 3 mittelbar über die Weichteile am Sitzbein 8, insbesondere am Sitzbeinhöcker 11, abstützt, wie dies insbesondere schematisch aus der Fig. 3 ersichtlich ist. Hierzu weist die obere Oberschenkelfassung 19, wie dies insbesondere aus der Fig. 4 und der Fig. 5 ersichtlich ist, am oberen Fassungsrand 20 einen in der Orthesen-Gebrauchsstellung 3 der Oberschenkelinnenseite zugeordneten gekrümmten Teilbereich 21 auf, der in Richtung zum Sitzbein 8 hin nach außen gekrümmt bzw. gewölbt ist und damit einen Sitzbein-Abstützbereich ausbildet.

Wie dies weiter aus der Zusammenschau der Fig. 4 und 5 ersichtlich ist, erstreckt sich die obere Oberschenkel-Fassung 19 am oberen Fassungsrand 20 mit einem der Oberschenkelaußenseite 22 zugeordneten Teilbereich 22 über den Bereich des großen Rollhügels 15 des Oberschenkelknochens 12 hinaus, wodurch sichergestellt ist, dass die obere Oberschenkel-Fassung 19 sich im Wesentlichen über die Weichteile am Sitzbein bzw. am Sitzbeinhöcker 11 abstützt.

Wie dies weiter aus den Fig. 1, 3, 4 und 5 ersichtlich ist, erstreckt sich die obere Oberschenkel-Fassung 19 vom oberen Fassungsrand 20 ausgehend mit einem zylinderförmigen Schaftbereich 23 bis über den Bereich des kleinen Rollhügels 24 (siehe Fig. 3) des Oberschenkelknochens 12 hinaus in den Bereich des Oberschenkelschafts 14 hinein.

Desweiteren weist die hier beispielhaft gezeigte erfindungsgemäße Hüftgelenkentlastungsorthese eine, bezogen auf die Orthesen-Gebrauchsstellung 3, untere, von der oberen Oberschenkelfassung 19 beabstandete Oberschenkel-Fassung 25 auf, die (vergleiche Fig. 3) den Oberschenkel im Übergangsbereich zum Kniegelenk 16 umgreift und sich in der gezeigten Orthesen-Gebrauchsstellung mittelbar über die Weichteile des Körpers am unteren verdickten Ende des Oberschenkel-Knochens 12, insbesondere an den beiden Femurkondylen 17, 18, abstützt, was nachfolgend noch näher erläutert wird.

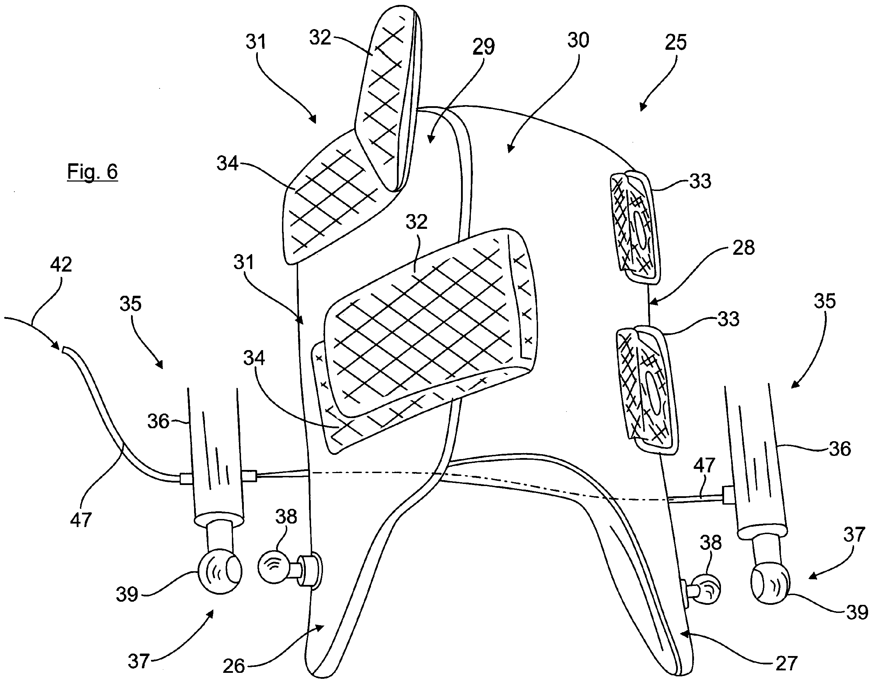

Wie dies insbesondere aus der Fig. 6 ersichtlich ist, ist die untere Oberschenkel-Fassung 25 als öffenbare Ringfassung ausgebildet, die bezogen auf die Orthesen-Gebrauchsstellung 3, zwei voneinander in Umfangsrichtung beabstandete sowie nach unten abragende lappenartige Fortsätze 26, 27 aufweist, die sich in der in der Fig. 3 gezeigten Orthesen-Gebrauchsstellung 3 mittelbar über die Weichteile jeweils an einem der zugeordneten Femurkondylen 17, 18 abstützen.

Wie dies weiter aus der Zusammenschau der Fig. 1, 3 und 6 ersichtlich ist, erstreckt sich die untere Oberschenkel-Fassung 25, bezogen auf die Orthesen-Gebrauchsstellung 3, von den beiden lappanartigen Fortsätzen 26, 27 ausgehend mit einem zylinderförmigen Schaftbereich 28 nach oben in Richtung zum mittleren Oberschenkelbereich.

Die untere Oberschenkel-Fassung 25 ist dabei als öffenbare Ringfassung ausgebildet, die im Bereich des zlyinderförmigen Schaftbereiches 28 sich in Umfangsrichtungsrichtung ggf. überlappende bzw. einander zumindest zugeordnete Teilbereiche 29, 30 aufweist, an denen hier beispielhaft zwei Klettverschlüsse 31 als Fixierelement angeordnet sind.

Die Klettverschlüsse 31 weisen jeweils ein an dem ersten Teilbereich 29 festgelegtes Klettband 32 auf, das durch eine am anderen, zweiten Teilbereich 30 festgelegte bzw. gelagerte Öse 33 hindurchführbar ist und zur Festlegung wieder zurück zu einem Befestigungsbereich 34 am ersten Teilbereich 29 geführt werden kann, um die untere Oberschenkel-Fassung 25 festzuzurren und festzulegen.

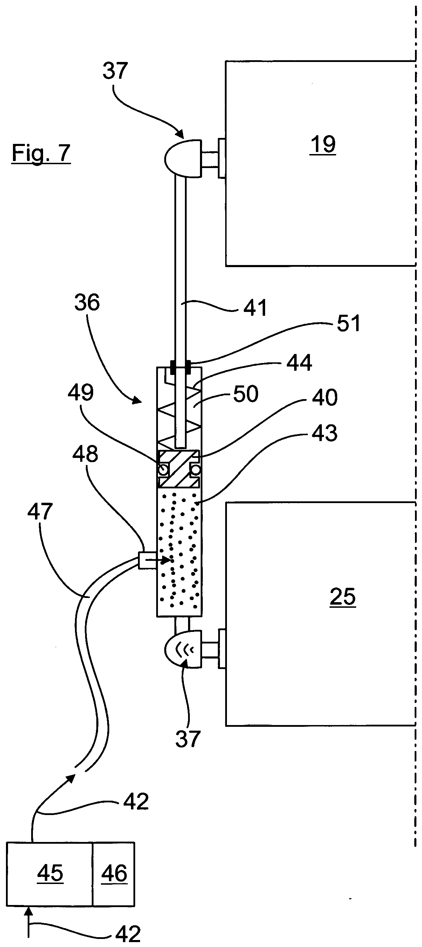

Wie dies aus den Figuren, und hier insbesondere aus der eine schematische Prinzipdarstellung zeigenden Fig. 7 ersichtlich ist, weist die Hüftgelenkentlastungsorthese 1 hier beispielhaft zwei Zylinder-Kolben-Einheiten 35 als Verstelleinrichtungen auf, die einander in Orthesen-Umfangsrichtung diametral gegenüberliegen.

Wie dargestellt, sind die Zylinder 36 der Zylinder-Kolben-Einheiten 35 optional an der unteren Oberschenkelfassung 25, vorzugsweise im Bereich deren lappenartiger Fortsätze 26, 27, gelenkig gelagert, und zwar bevorzugt mittels eines Kugelgelenkes bzw. einer Kugelgelenkanordnung 37, die hier wiederum beispielhaft eine fortsatzseitige Gelenkkugel 38 aufweist, die mit einer zylinderseitigen Gelenkpfanne 39 zusammenwirkt.

In analoger Weise sind alternativ oder zusätzlich die mit dem Kolben 40 (vergleiche Fig. 7) verbundenen Kolbenstangen 41 der Zylinder-Kolben-Einheiten 35 hier optional ebenfalls gelenkig an der oberen Oberschenkelfassung 19 gelagert, und zwar bevorzugt mittels analog ausgebildeter Kugelgelenke bzw. Kugelgelenkanordnungen 37 gelenkig an der oberen Oberschenkelfassung 19 gelagert.

Die die Verstelleinrichtungen ausbildenden Zylinder-Kolben-Einheiten 35 sind hier bevorzugt, wie in der Fig. 7 dargestellt, als einfach wirkende Pneumatikzylinder ausgebildet, bei denen das Arbeitsmedium, bevorzugt Luft 42, in einen Arbeitsraum 43 des Zylinders eingebracht wird, wodurch der Kolben 40 gegen die Kraft eines z.B. mechanischen Federelementes 44 in eine definiert vorgegebene Kolbenposition verlagert werden kann.

Mittels einer derartigen Verstelleinrichtung in Form der hier beispielhaften Zylinder-Kolben-Einheiten 35 lässt sich der Abstand zwischen der oberen Oberschenkel-Fassung 19 und der unteren Oberschenkel-Fassung 25 individuell und im Wesentlichen dauerhaft verändern und einstellen, so dass eine, eine definierte Hüftgelenkentlastung bewirkende Verspannkraft zwischen dem unteren verdickten Ende des Oberschenkelknochens 12 und dem Sitzbein 11 in der in der Fig. 3 gezeigten Orthesen-Gebrauchsstellung 3 einstellbar ist.

Zur Förderung des Arbeitsmediums Luft ist hier zum Beispiel eine Fördereinrichtung 45, zum Beispiel ein Kompressor oder dergleichen, vorgesehen, der Luft gesteuert bzw. geregelt mittels einer Steuer- und/oder Regeleinrichtung 46 über eine Mediumleitung 47, die über ein Anschlussventil 48 an den Zylinder 36 angeschlossen ist, in den Arbeitsraum 43 fördert. Die Verbindung zwischen der Mediumleitung 47 und dem Anschlussventil 48 kann dabei lösbar ausgebildet sein, um die jeweiligen Mediumleitungen 47, die zu den jeweiligen Zylindern 36 bzw. deren Arbeitsräumen 43 geführt sind, entfernen zu können, nachdem die gewünschte Verstellposition der Zylinder-Kolben-Einheiten 35 und damit der gewünschte Abstand zwischen beiden Oberschenkel-Fassungen 19 und 25 hergestellt ist.

Um die Gasdichtheit zwischen dem Arbeitsraum und einem diesen mit Bezug zum Kolben gegenüberliegenden Zylinderraum 50, in dem das mechanische Federelement 44 aufgenommen ist, herzustellen, kann der Kolben 40 mit einer Kolbendichtung 49 versehen sein.

Zudem kann der Zylinderraum 50 eine Be- und/oder Entlüftungsöffnung 51 aufweisen, um zu vermeiden, dass bei einer Verlagerung des Kolbens in der Bildebene der Fig. 7 nach oben ein Überdrück im Zylinderraum 50 herrscht. Die Be- und/oder Entlüftungsöffnung 51 ist hier gleichzeitig die Durchführöffnung der Kolbenstange 41, kann aber auch auf jede andere denkbare Art und Weise ausgebildet sein.

Wie in der Fig. 6 lediglich äußerst schematisch angedeutet, können die Zylinder-Kolben-Einheiten 35 bzw. deren Arbeitsräume 43 miteinander strömungstechnisch über entsprechende Mediumleitungen 47 verbunden sein, um die jeweiligen Arbeitsräume 43 mit Luft 42 als Arbeitsmedium zu beaufschlagen. Es versteht sich, dass selbstverständlich jeder Zylinder 36 der Zylinder-Kolben-Einheiten 35 auch unabhängig und separat angesteuert werden kann. Das in der Fig. 6 gezeigte Ausführungsbeispiel ist somit lediglich prinzipieller Natur und soll lediglich verdeutlichen, dass jeder Arbeitsraum 43 der Zylinder-Kolben-Einheiten 35 mit Luft als Arbeitsmedium zu beaufschlagen ist.

Die Oberschenkel-Fassungen 19, 25 sind bevorzugt aus einem harten Kunststoffmaterial, insbesondere aus einem faserverstärkten Kunststoffmaterial hergestellt, wobei auf der Innenseite eine Polsterung angebracht ist, insbesondere im Bereich der knochenseitigen Abstützpunkte Polsterpelotten vorgesehen bzw. angeordnet sein können.

## Patentansprüche

1. Hüftgelenkentlastungsorthese (1),
mit einer, bezogen auf die Orthesen-Gebrauchsstellung (3), oberen, den Oberschenkel wenigstens bereichsweise im Übergangsbereich zum Hüftgelenk (4) umgreifenden Oberschenkel-Fassung (19), die als Tubersitz ausgebildet ist und sich in der Orthesen-Gebrauchsstellung (3) mittelbar über die Weichteile am Sitzbein (8), insbesondere am Sitzbeinhöcker (11), abstützt,
**dadurch gekennzeichnet,**
**dass** eine, bezogen auf die Orthesen-Gebrauchsstellung (3), untere, von der oberen Oberschenkel-Fassung (19) beabstandete Oberschenkel-Fassung (25) vorgesehen ist, die den Oberschenkel im Übergangsbereich zum Kniegelenk (16) wenigstens bereichsweise umgreift und sich in der Orthesen-Gebrauchsstellung (3) mittelbar über die Weichteile am unteren verdickten Ende des Oberschenkelknochens (12), insbesondere an den beiden Femurkondylen (17, 18), abstützt, und
**dass** wenigstens eine Verstelleinrichtung (35) vorgesehen ist, die einerseits an der oberen Oberschenkel-Fassung (19) und andererseits an der unteren Oberschenkel-Fassung (25) angreift und mittels der, zur Einstellung einer, eine definierte Hüftgelenkentlastung bewirkenden Verspannkraft zwischen dem unteren verdickten Ende des Oberschenkelknochens (12) und dem Sitzbein (8) in der Orthesen-Gebrauchsstellung (3), der Abstand zwischen der oberen Oberschenkel-Fassung (19) und der unteren Oberschenkel-Fassung (25) veränderbar und einstellbar ist.

2. Hüftgelenkentlastungsorthese nach Anspruch 1, **dadurch gekennzeichnet, dass** die Verstelleinrichtung (35) eine Linearverstelleinrichtung ist.

3. Hüftgelenkentlastungsorthese nach Anspruch 2, **dadurch gekennzeichnet, dass** die Verstelleinrichtung (35) eine Zylinder-Kolben-Einheit mit einem, in einem Zylinder (36) geführten Kolben (40) sowie mitsamt dem Kolben (40) zugeordneter Kolbenstange (41) ist, wobei der Kolben (40), zu dessen Verlagerung in eine vorgegebene Abstützposition, mit einem Arbeitsmedium (42) beaufschlagbar ist, und
dass der Zylinder (36) mit einer ersten der beiden Oberschenkelfassungen (25) verbunden ist und die Kolbenstange (41) mit einer zweiten der beiden Oberschenkelfassungen (19) verbunden ist.

4. Hüftgelenkentlastungsorthese nach Anspruch 3, **dadurch gekennzeichnet, dass** die Zylinder-Kolben-Einheit eine einfach wirkende Verstelleinrichtung (35) ist, bei der das Arbeitsmedium (42) in einen Arbeitsraum (43) des Zylinders (36) einströmt und den Kolben (40) gegen die Kraft eines Federelementes (44) in eine definiert vorgegebene Kolbenposition verlagert.

5. Hüftgelenkentlastungsorthese nach Anspruch 4, **dadurch gekennzeichnet, dass** in einem dem Arbeitsraum (43) mit Bezug zum Kolben (40) gegenüberliegenden Zylinderraum (50) wenigstens eine Be- und/oder Entlüftungsöffnung (51) vorgesehen ist.

6. Hüftgelenkentlastungsorthese nach einem der Ansprüche 3 bis 5, **dadurch gekennzeichnet, dass** die Zylinder-Kolben-Einheit durch einen Hydraulikzylinder mit einem nicht-komprimierbaren Arbeitsmedium oder durch einen Pneumatikzylinder mit einem komprimierbaren Gas, insbesondere mit Luft, als Arbeitsmedium (42) gebildet ist.

7. Hüftgelenkentlastungsorthese nach einem der vorhergehenden Ansprüche, **dadurch gekennzeichnet, dass** mehrere Verstelleinrichtungen (35) vorgesehen sind, die in der Orthesen-Gebrauchsstellung (3) in Umfangsrichtung um den Oberschenkel herum voneinander beabstandet sind, insbesondere dergestalt, dass zwei in Umfangsrichtung diametral voneinander beabstandete Verstelleinrichtungen (35) vorgesehen sind.

8. Hüftgelenkentlastungsorthese nach einem der Ansprüche 4 bis 6 sowie 7, sofern in Abhängigkeit der Ansprüche 4-6, **dadurch gekennzeichnet, dass** der Zylinder (36) wenigstens ein Anschlusselement (48), insbesondere ein Anschlussventil, für eine Mediumleitung (47) aufweist, über das das Arbeitsmedium (42), bevorzugt gesteuert oder geregelt über eine Steuer- und/oder Regeleinrichtung, in den Arbeitsraum (43) einbringbar und/oder ausbringbar ist, wobei bevorzugt vorgesehen ist, dass die Mediumleitung (47) lösbar mit dem zylinderseitigen Anschlusselement (48) verbunden ist, das im abgekoppelten Zustand der Mediumleitung (47) dicht verschlossen ist.

9. Hüftgelenkentlastungsorthese nach einem der vorhergehenden Ansprüche, **dadurch gekennzeichnet, dass** die wenigstens eine Verstelleinrichtung (35) an der unteren und/oder oberen Oberschenkelfassung (19, 25) gelenkig, insbesondere mittels eines Kugelgelenkes (37), gelagert ist.

10. Hüftgelenkentlastungsorthese nach einem der vorhergehenden Ansprüche, **dadurch gekennzeichnet, dass** die obere Oberschenkelfassung (19) als, vorzugsweise geschlossene, Ringfassung ausgebildet ist, die sich, bezogen auf die Orthesen-Gebrauchsstellung (3), am oberen Fassungsrand (20) mit einem der Oberschenkelinnenseite zugeordneten Teilbereich (21) in Richtung zum Sitzbein (8) hin nach außen krümmt oder wölbt und einen Sitzbein-Abstützbereich ausbildet.

11. Hüftgelenkentlastungsorthese nach Anspruch 10, **dadurch gekennzeichnet, dass** sich die obere Oberschenkelfassung (19), bezogen auf die Orthesen-Gebrauchsstellung (3), am oberen Fassungsrand (20) mit einem der Oberschenkelaußenseite zugeordneten Teilbereich (22) über den Bereich des großen Rollhügels (15) des Oberschenkelknochens (12) hinaus erstreckt.

12. Hüftgelenkentlastungsorthese nach Anspruch 11, **dadurch gekennzeichnet, dass** sich die obere Oberschenkelfassung (19) vom, bezogen auf die Orthesen-Gebrauchsstellung (3), oberen Fassungsrand (20) ausgehend mit einem zylinderförmigen Schaftbereich (23) bis über den Bereich des kleinen Rollhügels (24) des Oberschenkelknochens (12) hinaus in den Bereich des Oberschenkelschaftes (14) erstreckt.

13. Hüftgelenkentlastungsorthese nach einem der vorhergehenden Ansprüche, **dadurch gekennzeichnet, dass** die untere Oberschenkelfassung (25) als, vorzugsweise öffenbare, Ringfassung ausgebildet ist, die, bezogen auf die Orthesen-Gebrauchsstellung (3), zwei voneinander in Umfangsrichtung beabstandete sowie nach unten abragende, lappenartige Fortsätze (26, 27) aufweist, die sich in der Orthesen-Gebrauchsstellung (3) mittelbar über die Weichteile jeweils an einem der Femurkondylen (17, 18) abstützen.

14. Hüftgelenkentlastungsorthese nach Anspruch 13, **dadurch gekennzeichnet, dass** zwei Verstelleinrichtungen (35) vorgesehen sind, von denen jeweils eine im Bereich eines der lappenartigen Fortsätze (26, 27) angreift und angebunden ist, insbesondere gelenkig angebunden ist und/oder sich im Wesentlichen geradlinig nach oben zu der, vorzugsweise gelenkigen, Anbindungsstelle an der oberen Oberschenkelfassung (19) erstreckt.

15. Hüftgelenkentlastungsorthese nach Anspruch 13 oder Anspruch 14, **dadurch gekennzeichnet, dass** sich die untere Oberschenkelfassung (25), bezogen auf die Orthesen-Gebrauchsstellung (3), von den beiden lappenartigen Fortsätzen (26, 27) ausgehend mit einem zylinderförmigen Schaftbereich (28) nach oben in Richtung zum mittleren Oberschenkelbereich erstreckt.

16. Hüftgelenkentlastungsorthese nach Anspruch 15, **dadurch gekennzeichnet, dass** die untere Oberschenkelfassung (25) als öffenbare Ringfassung ausgebildet ist, die sich im Bereich des zylinderförmigen Schaftbereiches (28) in Umfangsrichtung gegebenenfalls überlappende und/oder einander zugeordnete Teilbereiche (29, 30) aufweist, an denen wenigstens ein Fixierelement angeordnet ist.

17. Hüftgelenkentlastungsorthese nach Anspruch 16, **dadurch gekennzeichnet, dass** das wenigstens eine Fixierelement eine Klettverschlussanordnung (31) ist, die ein an einem ersten Teilbereich (29) festgelegtes Klettband (32) aufweist, das durch eine am anderen zweiten Teilbereich (30) gelagerte Öse (33) hindurchführbar und zur Festlegung wieder zurück zu einem Befestigungsbereich (34) am ersten Teilbereich (29) führbar ist.

## Claims

1. Hip joint relief orthosis (1),
having an upper, with regard to the orthosis use position (3), thigh cuff (19) that engages around the thigh at least regionally in the transition region to the hip joint (4), is in the form of a tuberosity support and, in the orthosis use position (3), is supported indirectly via the soft tissue on the ischium (8), in particular on the ischial tuberosity (11),
**characterized**
**in that** a lower, with regard to the orthosis use position (3), thigh cuff (25) that is spaced apart from the upper thigh cuff (19) is provided, said lower thigh cuff (25) engaging around the thigh at least regionally in the transition region to the knee joint (16) and, in the orthosis use position (3), being supported indirectly via the soft tissue on the lower enlarged end of the femur (12), in particular on the two femur condyles (17, 18), and
**in that** at least one adjusting device (35) is provided, which acts on the upper thigh cuff (19) on one side and on the lower thigh cuff (25) on the other side and by means of which, in order to set a bracing force, effecting defined hip joint relief, between the lower enlarged end of the femur (12) and the ischium (8) in the orthosis use position (3), the distance between the upper thigh cuff (19) and the lower thigh cuff (25) is variable and settable.

2. Hip joint relief orthosis according to Claim 1, **characterized in that** the adjusting device (35) is a linear adjusting device.

3. Hip joint relief orthosis according to Claim 2, **characterized in that** the adjusting device (35) is a cylinder-piston unit having a piston (40) guided in a cylinder (36) and together therewith a piston rod (41) assigned to the piston (40), wherein, in order to be moved into a predetermined support position, the piston (40) is able to be acted on by a working medium (42), and
**in that** the cylinder (36) is connected to a first of the two thigh cuffs (25) and the piston rod (41) is connected to a second of the two thigh cuffs (19).

4. Hip joint relief orthosis according to Claim 3, **characterized in that** the cylinder-piston unit is a single-acting adjusting unit (35), in which the working medium (42) flows into a working chamber (43) of the cylinder (36) and moves the piston (40) into a predefined piston position counter to the force of a spring element (44).

5. Hip joint relief orthosis according to Claim 4, **characterized in that** at least one ventilation and/or venting opening (51) is provided in a cylinder chamber (50) located opposite the working chamber (43) with regard to the piston (40).

6. Hip joint relief orthosis according to one of Claims 3 to 5, **characterized in that** the cylinder-piston unit is formed by a hydraulic cylinder with a non-compressible working medium or by a pneumatic cylinder with a compressible gas, in particular with air, as working medium (42).

7. Hip joint relief orthosis according to one of the preceding claims, **characterized in that** a plurality of adjusting devices (35) are provided, which are spaced apart from one another in the circumferential direction around the thigh in the orthosis use position (3), in particular such that two adjusting devices (35) that are spaced apart diametrically from one another in the circumferential direction are provided.

8. Hip joint relief orthosis according to one of Claims 4 to 6 and 7, where dependent on Claims 4-6, **characterized in that** the cylinder (36) has at least one connection element (48), in particular a connection valve, for a medium line (47), the working medium (42) being able to be introduced into and/or discharged from the working chamber (43), preferably in a manner controlled or regulated via a control and/or regulating device, via said connection element (48), wherein provision is preferably made for the medium line (47) to be connected releasably to the cylinder-side connection element (48), which is tightly closed in the uncoupled state of the medium line (47).

9. Hip joint relief orthosis according to one of the preceding claims, **characterized in that** the at least one adjusting device (35) is mounted on the lower and/or upper thigh cuff (19, 25) in an articulated manner, in particular by means of a ball joint (37).

10. Hip joint relief orthosis according to one of the preceding claims, **characterized in that** the upper thigh cuff (19) is in the form of a, preferably closed, ring cuff which, with regard to the orthosis use position (3), is curved or bulges outwardly in the direction of the ischium (8) at the upper cuff rim (20) with a subregion (21) assigned to the thigh inner side and forms an ischial support region.

11. Hip joint relief orthosis according to Claim 10, **characterized in that** the upper thigh cuff (19) extends, with regard to the orthosis use position (3), beyond the region of the greater trochanter (15) of the femur (12) at the upper cuff rim (20) with a subregion (22) assigned to the thigh outer side.

12. Hip joint relief orthosis according to Claim 11, **characterized in that** the upper thigh cuff (19) extends with a cylindrical shank region (23) from the upper, with regard to the orthosis use position (3), thigh cuff (20) to beyond the region of the lesser trochanter (24) of the femur (12) and into the region of the femoral shaft (14).

13. Hip joint relief orthosis according to one of the preceding claims, **characterized in that** the lower thigh cuff (25) is in the form of a, preferably openable, ring cuff which, with regard to the orthosis use position (3), has two lobe-like extensions (26, 27) which are spaced apart from one another in the circumferential direction and project downwards and which, in the orthosis use position (3), are each supported indirectly via the soft tissue on one of the femur condyles (17, 18).

14. Hip joint relief orthosis according to Claim 13, **characterized in that** two adjusting devices (35) are provided, in each case one of which acts and is attached, in particular attached in an articulated manner, in the region of one of the lobe-like extensions (26, 27) and/or extends substantially rectilinearly upwards as far as the, preferably articulated, attachment point on the upper thigh cuff (19).

15. Hip joint relief orthosis according to Claim 13 or Claim 14, **characterized in that** the lower thigh cuff (25) extends, with regard to the orthosis use position (3), upwards with a cylindrical shank region (28) from the two lobe-like extensions (26, 27) in the direction of the middle thigh region.

16. Hip joint relief orthosis according to Claim 15, **characterized in that** the lower thigh cuff (25) is in the form of an openable ring cuff which, in the region of the cylindrical shank region (28), has subregions (29, 30) which optionally overlap and/or are assigned to one another in the circumferential direction and at which at least one fixing element is arranged.

17. Hip joint relief orthosis according to Claim 16, **characterized in that** the at least one fixing element is a hook-and-loop arrangement (31) which has a hook-and-loop tape (32) that is fastened to a first subregion (29), is able to be passed through an eye (33) mounted in the other, second subregion (30) and, in order to be fastened, is able to be guided back again to a fastening region (34) in the first subregion (29).

## Revendications

1. Orthèse de décharge de la hanche (1), comprenant une gaine fémorale supérieure (19) par rapport à la position d'utilisation de l'orthèse (3), venant en prise autour de la cuisse au moins en partie dans la région de transition avec l'articulation de la hanche (4), qui est réalisée sous forme de siège de tubérosité et qui s'appuie, dans la position d'utilisation de l'orthèse (3), indirectement par le biais des parties molles, contre l'ischion (8), en particulier au niveau des tubérosités ischiatiques (11), **caractérisée en ce**
**qu'**il est prévu une gaine fémorale inférieure (25) par rapport à la position d'utilisation de l'orthèse (3), espacée de la gaine fémorale supérieure (19), qui vient en prise au moins en partie autour de la cuisse dans la région de transition avec l'articulation du genou (16) et qui s'appuie, dans la position d'utilisation de l'orthèse (3), indirectement par le biais des parties molles, contre l'extrémité inférieure épaissie de la tête fémorale (12), en particulier contre les deux condyles du fémur (17, 18), et
en ce qu'au moins un dispositif de réglage (35) est prévu, lequel vient en prise d'une part avec la gaine fémorale supérieure (19) et d'autre part avec la gaine fémorale inférieure (25), et au moyen duquel, pour l'ajustement d'une force de serrage causant une décharge définie de l'articulation fémorale entre l'extrémité inférieure épaissie de la tête fémorale (12) et l'ischion (8) dans la position d'utilisation de l'orthèse (3), la distance entre la gaine fémorale supérieure (19) et la gaine fémorale inférieure (25) peut être modifiée et ajustée.

2. Orthèse de décharge de la hanche selon la revendication 1, **caractérisée en ce que** le dispositif de réglage (35) est un dispositif de réglage linéaire.

3. Orthèse de décharge de la hanche selon la revendication 2, **caractérisée en ce que** le dispositif de réglage (35) est une unité cylindre-piston comprenant un piston (40) guidé dans un cylindre (36) ainsi qu'une tige de piston (41) associée au piston (40), le piston (40), en vue de son déplacement dans une position de support prédéfinie, pouvant être sollicité avec un fluide de travail (42), et
**en ce que** le cylindre (36) est connecté à une première des deux gaines fémorales (25) et la tige de piston (41) est connectée à une deuxième des deux gaines fémorales (19).

4. Orthèse de décharge de la hanche selon la revendication 3, **caractérisée en ce que** l'unité cylindre-piston est un dispositif de réglage (35) à fonctionnement simple, dans lequel le fluide de travail (42) afflue à l'intérieur d'un espace de travail (43) du cylindre (36) et déplace le piston (40) à l'encontre de la force d'un élément de ressort (44) dans une position de piston préétablie de manière définie.

5. Orthèse de décharge de la hanche selon la revendication 4, **caractérisée en ce qu**'au moins une ouverture de ventilation et/ou de désaérage (51) est prévue dans un espace de cylindre (50) opposé à l'espace de travail (43) par rapport au piston (40).

6. Orthèse de décharge de la hanche selon l'une quelconque des revendications 3 à 5, **caractérisée en ce que** l'unité cylindre-piston est formée par un cylindre hydraulique comprenant un fluide de travail non compressible ou par un cylindre pneumatique comprenant un gaz compressible, en particulier de l'air, en tant que fluide de travail (42).

7. Orthèse de décharge de la hanche selon l'une quelconque des revendications précédentes, **caractérisée en ce que** plusieurs dispositifs de réglage (35) sont prévus, lesquels sont espacés les uns les autres dans la position d'utilisation de l'orthèse (3), dans la direction périphérique autour de la cuisse, en particulier de telle sorte que deux dispositifs de réglage (35) espacés diamétralement l'un de l'autre dans la direction périphérique soient prévus.

8. Orthèse de décharge de la hanche selon l'une quelconque des revendications 4 à 6, et 7 dans la mesure où elle dépend des revendications 4 à 6, **caractérisée en ce que** le cylindre (36) présente au moins un élément de raccordement (48), en particulier une soupape de raccordement pour une conduite de fluide (47), par le biais duquel le fluide de travail (42), de préférence commandé ou régulé par le biais d'un dispositif de commande et/ou de régulation, peut être introduit et/ou évacué dans ou hors l'espace de travail (43), de préférence la conduite de fluide (47) étant connectée de manière détachable à l'élément de raccordement du côté du cylindre (48) qui est fermé hermétiquement dans l'état désaccouplé de la conduite de fluide (47).

9. Orthèse de décharge de la hanche selon l'une quelconque des revendications précédentes, **caractérisée en ce que** l'au moins un dispositif de réglage (35) est supporté de manière articulée, en particulier au moyen d'une articulation sphérique (37) à la gaine fémorale inférieure et/ou supérieure (19, 25).

10. Orthèse de décharge de la hanche selon l'une quelconque des revendications précédentes, **caractérisée en ce que** la gaine fémorale supérieure (19) est réalisée sous forme de gaine annulaire de préférence fermée qui, par rapport à la position d'utilisation de l'orthèse (3), est courbée ou cintrée vers l'extérieur au niveau du bord supérieur de la gaine (20) avec une région partielle (21) associée au côté intérieur de la cuisse dans la direction de l'ischion (8) et constitue une région de support de l'ischion.

11. Orthèse de décharge de la hanche selon la revendication 10, **caractérisée en ce que** la gaine fémorale supérieure (19), par rapport à la position d'utilisation de l'orthèse (3), s'étend au niveau du bord supérieur de la gaine (20) avec une région partielle (22) associée au côté extérieur de la cuisse, au-delà de la région du grand trochanter (15) de la tête fémorale (12).

12. Orthèse de décharge de la hanche selon la revendication 11, **caractérisée en ce que** la gaine fémorale supérieure (19), partant du bord supérieur de la gaine (20), par rapport à la position d'utilisation de l'orthèse (3), s'étend avec une région de tige cylindrique (23) jusqu'au-delà de la région du petit trochanter (24) de la tête fémorale (12) dans la région de la tige fémorale (14).

13. Orthèse de décharge de la hanche selon l'une quelconque des revendications précédentes, **caractérisée en ce que** la gaine fémorale inférieure (25) est réalisée sous forme de gaine annulaire de préférence pouvant être ouverte, qui, par rapport à la position d'utilisation de l'orthèse (3), présente deux saillies (26, 27) de type languettes, espacées l'une de l'autre dans la direction périphérique et faisant saillie vers le bas, qui s'appuient, dans la position d'utilisation de l'orthèse (3), indirectement par le biais des parties molles à chaque fois contre l'un des condyles du fémur (17, 18).

14. Orthèse de décharge de la hanche selon la revendication 13, **caractérisée en ce que** deux dispositifs de réglage (35) sont prévus, dont à chaque fois l'un s'engage et est raccordé, en particulier de manière articulée, dans la région de l'une des saillies (26, 27) de type languettes et/ou s'étend essentiellement en ligne droite vers le haut jusqu'à la position de raccordement de préférence articulée à la gaine fémorale supérieure (19).

15. Orthèse de décharge de la hanche selon la revendication 13 ou la revendication 14, **caractérisée en ce que** la gaine fémorale inférieure (25), par rapport à la position d'utilisation de l'orthèse (3), s'étend depuis les deux saillies (26, 27) en forme de languettes, avec une région de tige cylindrique (28) vers le haut dans la direction de la région fémorale centrale.

16. Orthèse de décharge de la hanche selon la revendication 15, **caractérisée en ce que** la gaine fémorale inférieure (25) est réalisée sous forme de gaine annulaire pouvant être ouverte qui présente des régions partielles (29, 30) associées l'une à l'autre et/ou se chevauchant éventuellement dans la région de la région de tige cylindrique (28) dans la direction périphérique.

17. Orthèse de décharge de la hanche selon la revendication 16, **caractérisée en ce que** l'au moins un élément de fixation est un système de fermeture de type Velcro (31) qui présente une bande de type Velcro (32) fixée au niveau d'une première région partielle (29), qui peut être guidée à travers un oeillet (33) supporté sur l'autre deuxième région partielle (30) et qui peut être ramenée pour la fixation vers une région de fixation (34) au niveau de la première région partielle (29).
